# EUROPEAN PATENT APPLICATION

(11) **EP 1 832 873 A1**
(43) Date of publication of application: **12.09.2007**
(21) Application number: 07003144.8
(22) Date of filing: 14.02.2007
(51) Int. Cl.: G01N 27/49, G01N 33/00

(54) **Electrochemical gas sensor with reduced wake-up time**

(30) Priority: 16.02.2006 US 355793
(71) Applicant: Invensys Controls UK Limited, Bressenden Place London, SW1E 5BF (GB)
(72) Inventor: Herbert, Andrew G., Stoke Gabriel, Totnes TQ9 6SJ (GB)
(74) Representative: Friese, Martin

(57) **Abstract**

An electrochemical gas sensor that includes a wick (54) held in contact with an electrode substrate having at least a working electrode (44) and a counter electrode (46) by a spreader plate (66). The spreader plate (66) is formed as a planar element that includes a series of perforations (74) to reduce the rigidity of the spreader plate. The perforated spreader plate is held in contact with the wick by a pair of spring arms (70) to bias the wick into contact with the electrodes formed on the substrate.

## Description

### BACKGROUND OF THE INVENTION

The present invention relates to an electrochemical gas sensor. More specifically, the present invention relates to an electrochemical gas sensor that includes an improved spreader plate to enhance the physical contact between the wick and electrode within the gas sensor to reduce the wake-up time of the sensor.

An electrochemical gas sensor for sensing an oxidizable or reducible gas (e.g. carbon monoxide) in the atmosphere usually contains a sensing or working electrode, a counter electrode, a reference electrode and an inlet (usually a diffusion barrier) to allow the atmosphere to permeate to the working electrode. Both the working electrode and the counter electrode are in contact with an electrolyte in order to produce an electrochemical reaction at the working electrode with the gas to be sensed and secondly to produce an electrochemical reaction at the counter electrode with oxygen in the atmosphere, electrolyte or other gas source. Current is carried through the electrolyte solution by ions produced in the reaction and by electrons through an external circuit, where the current in the circuit indicates the gas concentration level. The reference electrode is employed in combination with a potentiostat circuit to maintain the potential between the working electrode and the cell electrolyte in order to increase the stability of operation.

In terms of physical construction, the sensor normally comprises an external housing that acts as a reservoir for the electrolyte. The sensor typically includes a wick having a portion immersed in the electrolyte and a portion in contact with the electrode assembly such that the electrodes formed as part of the electrode assembly are immersed in the electrolyte. The gas sensor includes external electrical terminals that allow for electrical connection with the electrodes included in the electrochemical gas sensor.

In the design of prior art electrochemical sensors, the wick is pressed into contact with a porous, flexible substrate that includes the working, reference and counter electrodes. Specifically, a spreader plate is positioned in contact with the wick to spread the wick out and hold the wick in contact with the electrode substrate. Typically, the spreader plate is held in contact with the wick by a spring arm assembly formed as part of the sensor body.

Although the spreader plate presses the wick into contact with the electrodes, the substrate used to support the three electrodes is a hydrophobic element. Thus, when the wick is wetted with the electrolyte, the wick has a stronger affinity to the spreader plate than the electrode substrate, which can result in the separation of the wick from the electrode substrate. The separation between the wick and the electrode substrate can result in the electrochemical gas sensor taking up to 21 days to "wake-up" after the initial manufacture of the electrochemical sensor. Since the electrochemical gas sensors are typically formed in large quantities, a 21 day delay from the manufacture of the sensor to initial testing may result in a significant volume of production prior to sensor testing.

Therefore, a need exists for an improved electrochemical sensor that includes a spreader plate that more effectively holds the wick in contact with the electrode substrate to decrease the wake-up time for the sensor.

### SUMMARY OF THE INVENTION

The present invention is an electrochemical gas sensor that reduces the wake-up time of the sensor. The electrochemical gas sensor, which can detect various different types of gases such as carbon monoxide, includes a housing that defines a reservoir for receiving a supply of an electrolyte. An electrode substrate extends across the top of the housing and includes at least a working electrode and a counter electrode each coupled to contact pins extending from the gas sensor housing. A wick is positioned within the housing and has side portions that extend into the reservoir to contact the electrolyte such that the electrolyte is absorbed by the wick and wets the electrodes formed on the electrode substrate. The wick is biased into contact with the electrodes formed on the substrate by a spreader plate positioned between the wick and a spring clip formed as part of the gas sensor housing. The spring clip exerts a bias force onto the spreader plate such that the spreader plate presses the wick into contact with the electrodes formed on the electrode substrate.

The spreader plate is formed from a polycarbonate material that is defined by an outer edge surface. The spreader plate includes a series of perforations that extend through the thickness of the spreader plate and are contained within the outer edge defining the spreader plate. The perforations formed in the spreader plate reduce the overall rigidity of the spreader plate and reduce the flexing of the spreader plate as a result of the bias force exerted by the spring clip. The reduction in the flexing of the spreader plate allows the spreader plate to more effectively hold the wick in contact with the lower surface of the electrode substrate, thereby increasing the wetting of the electrode formed on the substrate by the electrolyte. The increased contact between the wick and the electrodes formed on the substrate reduces the amount of time required for the electrochemical gas sensor to "wake-up" as compared to a gas sensor including a solid spreader plate.

The spreader plate can include any number of perforations spaced across the surface area of the spreader plate and formed in any configuration. The perforations not only reduce the rigidity of the spreader plate, but the perforations also allow gas to permeate through the spreader plate and into contact with both the wick and the substrate.

### BRIEF DESCRIPTION OF THE DRAWINGS

The drawings illustrate the best mode presently contemplated of carrying out the invention. In the drawings:

Fig. 1 is a front perspective view of an electrochemical gas sensor;

Fig. 2 is an exploded perspective view of the electrochemical gas sensor incorporating the spreader plate of the present invention;

Fig. 3 is a top view of a prior art spreader plate;

Fig. 4 is a top view of the spreader plate of the invention;

Fig. 5 is a section view of the electrochemical gas sensor; and

Fig. 6 is a magnified section view of the electrochemical gas sensor illustrating the position of the spreader plate relative to the wick and electrode assembly.

### DETAILED DESCRIPTION OF THE INVENTION

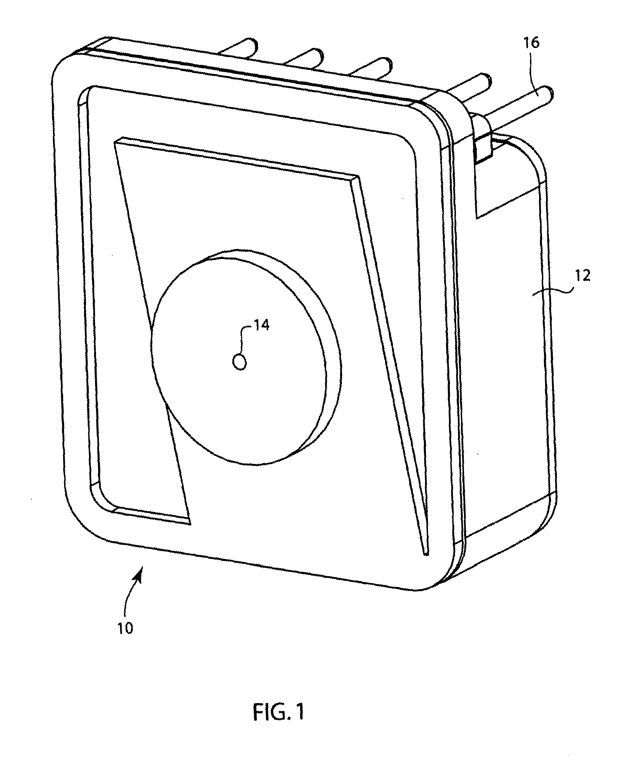

Fig. 1 illustrates an electrochemical gas sensor that is operable to detect the level of a gas, such as carbon monoxide, in the area surrounding the sensor. The sensor 10 includes an outer housing 12 that includes a gas aperture 14 that allows atmospheric gas to enter into the otherwise enclosed housing 12 for sensing. The outer housing 12 includes a series of pins 16 that allow the gas sensor 10 to be mounted to a conventional circuit board as part of a gas sensing device, such as a carbon monoxide detector. Each of the pins 16 is an electrical connection with an electrode formed within the electrochemical gas sensor 10 as will be described in greater detail below. In the preferred embodiment of the invention shown in Fig. 1, the entire housing 12 is formed from a durable plastic material, such as polypropylene.

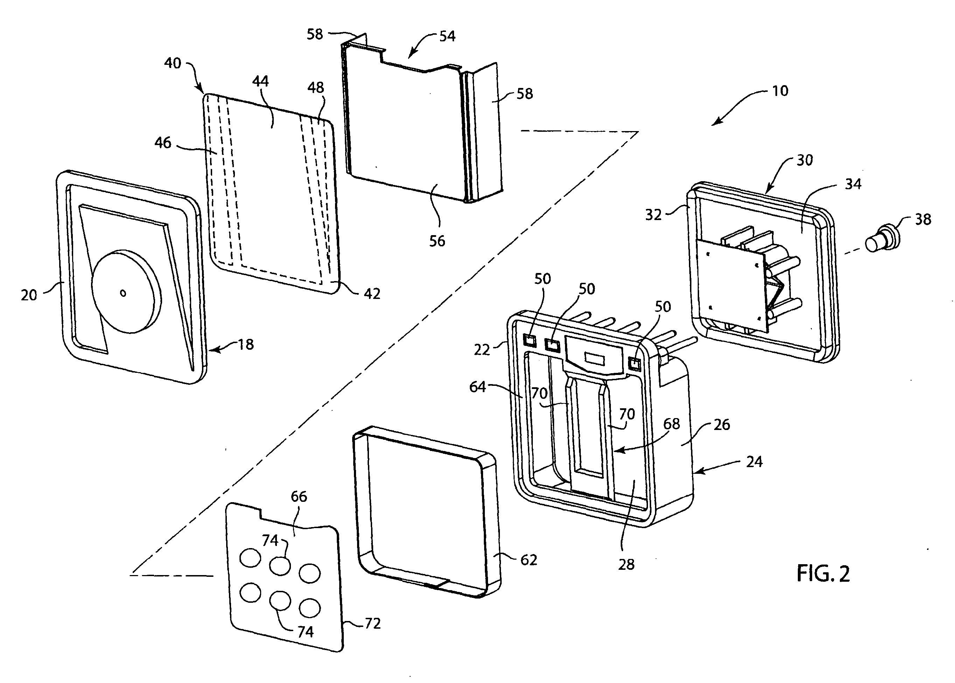

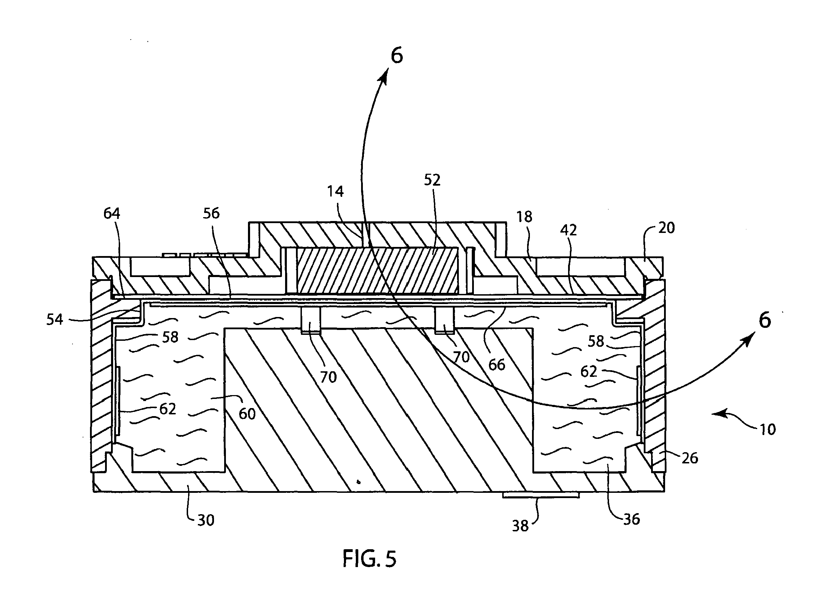

Fig. 2 illustrates an exploded view of the electrochemical gas sensor 10 constructed in accordance with the present invention. As shown in Fig. 2, the electrochemical gas sensor includes a cap member 18 having an outer edge surface 20 received within an outer flange 22 formed as part of a main body 24. The main body 24 includes an outer wall 26 defining an open interior 28. The main body 24 receives a cell lid subassembly 30 having an outer flange 32 received within the outer wall 26 of the main body 24 in a sealing relationship. The combination of the outer wall 26 and the bottom wall 34 of the cell lid subassembly 30 define a reservoir 36 for receiving a supply of an electrolyte when the electrochemical gas sensor 10 is in the assembled condition, as shown in Fig. 5. The cell lid subassembly 30 receives a filler plug 38 that is positioned within a hole in the subassembly after the reservoir 36 has been filled with the electrolyte solution.

Referring back to Fig. 2, the electrochemical gas sensor includes an electrode assembly 40 that includes a generally planar substrate 42 having a first surface and a second surface. In the embodiment of the invention shown in Fig. 2, the first surface faces the cap member 18, while the second surface includes a working electrode 44, a counter electrode 46 and a reference electrode 48. The electrodes 42, 44 and 46 are formed as a mixture of electrically conductive catalyst particles in PTFE binder and are screen printed or filter deposited onto the second surface of the substrate 42. Each of the electrodes 44, 46 and 48 are in contact with the pin contacts 50 formed as part of the main body 24.

As can be seen in Fig. 5, when the electrochemical gas sensor 10 is in the assembled position, a filter 52 is positioned between the cap member 18 and the electrode substrate 42. The filter 52 allows atmospheric gases to enter into the gas sensor through the gas aperture 14 and be brought into contact with the working electrode in a known manner.

Referring back to Fig. 2, the electrochemical gas sensor 10 includes a wick 54 that is formed from a porous felt material. The wick 54 includes a planar contact portion 56 and a pair of side flaps 58. When the wick 54 is positioned within the assembled gas sensor 10 shown in Fig. 5, the pair of side flaps 58 extend into the reservoir 36 and contact the supply of electrolyte 60 received within the reservoir 36. Since the wick 54 is formed from a hydrophilic material, the electrolyte 60 saturates the entire wick, including the planar contact portion 56 that is in contact with the electrodes formed on the second surface of the substrate 42. As illustrated in Figs. 2 and 5, the gas sensor 10 includes a wick retainer 62 that extends around the outer edges of the reservoir 36 and flexes outward into contact with the outer wall 26 to hold the side flaps 58 of the wick in contact with the outer walls 26. Preferably, the wick retainer 62 is formed from a flexible plastic material.

As can be understood in Figs. 2 and 5, the electrode subassembly 40 is sized to rest upon a support lip 64 and be entrapped between the support lip 64 and the cap member 18. The support lip 64 includes the series of pin contacts 50 as shown in Fig. 2.

When the gas sensor 10 is assembled as shown in Fig. 5, a spreader plate 66 is positioned in contact with the wick 54 and is biased into the position shown in Fig. 5 by a spring clip 68 having a pair of bias arms 70. As illustrated in Fig. 5, each of the bias arms 70 exert a physical force onto the spreader plate 66 to press the spreader plate 66, and thus the contact portion 56 of the wick 54, into contact with electrodes formed on the second surface of the substrate 42.

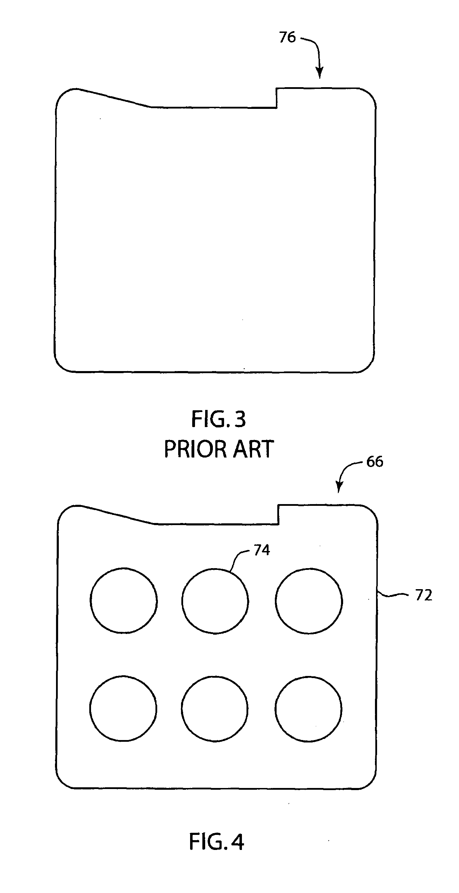

Referring now to Fig. 4, the spreader plate 66 is a generally planar element having an outer edge 72 that defines a generally rectangular element, as illustrated. The spreader plate 66 includes a plurality of perforations 74 that extend through the entire thickness of the spreader plate. In the embodiment illustrated in Fig. 4, each of the perforations 74 are formed as circular members equally spaced from each other. In the embodiment illustrated, each of the perforations 74 has a diameter of 5 millimeters while the spreader plate 66 has a height of 21.7 millimeters and a length of 24.0 millimeters. Although specific dimensions are listed for the spreader plate 66 and the perforations 74, it should be understood that various other dimensional measurements could be utilized while operating within the scope of the present invention.

In prior art electrochemical gas sensors, such as the Mark IV sensor sold by Monox, the spreader plate 76 is a solid member formed from a sheet of polycarbonate material, as shown in Fig. 3. When the prior art spreader plate 76 is utilized in a sensor similar to the embodiment shown in Fig. 5, the solid spreader plate had a tendency to pull the wick 54 toward the spreader plate by surface tension, thus moving the wick away from the electrodes formed on the second surface of the substrate 42. Since the wick 54 is pulled toward the prior art spreader plate, the amount of time required to wet the electrodes with electrolyte, commonly referred to as the wake-up time, was oftentimes as great as 21 days. The separation between the wick and the electrode substrate was further worsened by the stiffness of the prior art spreader plate 76, since the bias arms of the spring clip caused the outer edges of the spreader plate to bow outward, away from the electrode substrate 42. Further, since the prior art spreader plate 76 is a solid, polycarbonate sheet, gases that have diffused through the electrode assembly are unable to pass through the spreader plate and into the main cell volume.

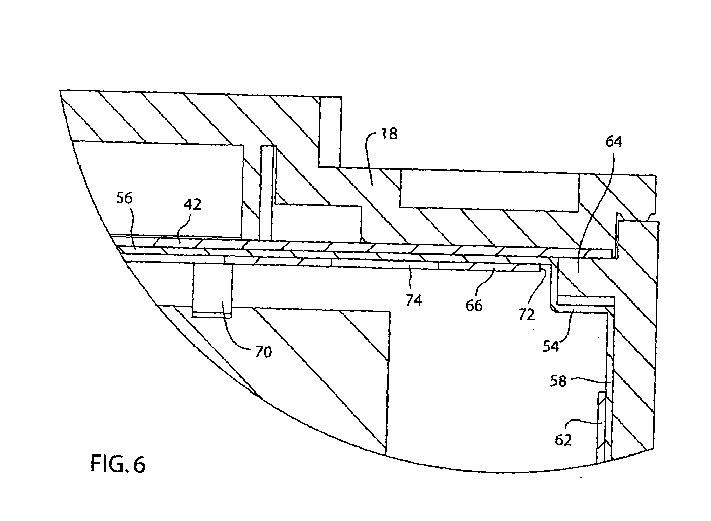

As shown in Figs. 4 and 6, the individual perforations 74 formed in the spreader plate 66 of the present invention allow the spreader plate to be less rigid as compared to the solid, prior art spreader plate 76 shown in Fig. 3. Thus, when the bias arms 70 of the spring clip 68 are brought into contact with the spreader plate 66, the spreader plate is less rigid and resists the tendency to bow downward away from the substrate 42. Further, the series of perforations contained within the polycarbonate spreader plate 66 allows gases contained within the open body of the gas sensor to permeate through the spreader plate 66 and into contact with the wick 54 and the electrode substrate 42.

In gas sensors in which the prior art, solid spreader plate 76 shown in Fig. 3 is replaced with the perforated spreader plate 66 shown in Fig. 4, the wake-up time for the electrochemical gas sensor is decreased from 21 days to less than 2 days. As an example, the electrochemical gas sensor 10 incorporating the spreader plate 66 has a yield rate of approximately 80% in less than 24 hours after the reservoir is filled with the electrolyte. Prior designs that include the solid spreader plate 76 have a yield rate of only about 12.5% during the same 24 hour period. Thus, replacing the solid spreader plate 76 with the perforated spreader plate 66 of Fig. 4 reduces the wake-up time of the gas sensor significantly.

Although the preferred embodiment of the invention is shown with circular perforations 76 spaced evenly throughout the surface area of the spreader plate 66, it should be understood that different shaped perforations and different spacing/sizes of the perforation can be utilized while operating within the scope of the present invention. The perforations function to reduce the stiffness of the polycarbonate spreader plate while also allowing gas to permeate through the spreader plate itself. Thus, different size perforations and configuration of the actual perforation itself can be utilized while operating within the scope of the present invention.

Accordingly the invention also relates to an electrochemical gas sensor that includes a wick held in contact with an electrode substrate having at least a working electrode and a counter electrode by a spreader plate. The spreader plate is formed as a planar element that includes a series of perforations to reduce the rigidity of the spreader plate. The perforated spreader plate is held in contact with the wick by a pair of spring arms to bias the wick into contact with the electrodes formed on the substrate.

## Claims

1. An electrochemical gas sensor, comprising:
a housing having a reservoir for receiving an electrolyte;
a substrate extending across the housing and including at least a working electrode and a counter electrode;
a wick positioned within the housing and extending into the reservoir to contact the electrolyte, the wick being in contact with the working and counter electrodes of the substrate; and
a spreader plate positioned within the housing and in contact with the wick to hold the wick in contact with the substrate, wherein the spreader plate is a planar member having a plurality of perforations formed therein.

2. An electrochemical gas sensor, comprising:
a housing having a reservoir for receiving an electrolyte;
an electrode assembly positioned within the housing and including at least a working electrode and a counter electrode;
a wick positioned within the housing and extending into the reservoir to contact the electrolyte, the wick being in contact with the working and counter electrodes; and
a spreader plate positioned within the housing and in contact with the wick to hold the wick in contact with the electrode assembly, wherein the spreader plate is a planar member having a plurality of perforations formed therein.

3. The gas sensor of any of the preceding claims, wherein the spreader plate contacts the wick to define a generally planar contact portion of the wick, wherein the contact portion of the wick contacts the working and counter electrodes.

4. The gas sensor of any of the preceding claims, wherein the spreader plate contacts the wick to define a generally planar contact portion of the wick, wherein the contact portion of the wick is adjacent the working and counter electrodes.

5. The gas sensor of any of the preceding claims, wherein the plurality of perforations are each circular.

6. The gas sensor of any of the preceding claims, further comprising a spring clip extending across the housing and positioned in contact with the spreader plate to hold the spreader plate in contact with the substrate.

7. The gas sensor of any of the preceding claims, further comprising a spring clip extending across the housing and positioned in contact with the spreader plate to hold the spreader plate in contact with the electrode assembly.

8. The gas sensor of any of the preceding claims, wherein the spreader plate includes an outer edge and wherein each of the plurality of perforations is positioned entirely within the outer edge.

9. The gas sensor of any of the preceding claims, wherein the contact portion of the wick is positioned between the spreader plate and the substrate.

10. The gas sensor of any of the preceding claims, wherein the contact portion of the wick is positioned between the spreader plate and the electrode assembly.

11. The gas sensor of any of the preceding claims, wherein the spreader plate is formed from polycarbonate.

12. The gas sensor of any of the preceding claims, wherein the spreader plate is positioned between the reservoir and the contact portion of the wick.
